(19) Europäisches Patentamt
European Patent Office
Office européen des brevets

(11) Veröffentlichungsnummer: **0 034 248**
**B1**

(12) # EUROPÄISCHE PATENTSCHRIFT

(45) Veröffentlichungstag der Patentschrift: **25.07.84**

(51) Int. Cl.$^3$: **C 07 J 9/00**

(21) Anmeldenummer: **81100145.2**

(22) Anmeldetag: **10.01.81**

(54) **Neue mehrfach ungesättigte BNC-Halogenide und Verfahren zu ihrer Herstellung.**

(30) Priorität: **15.01.80 DE 3001222**
**04.02.80 AT 582/80**
**19.02.80 US 122397**

(43) Veröffentlichungstag der Anmeldung: **26.08.81 Patentblatt 81/34**

(45) Bekanntmachung des Hinweises auf die Patenterteilung: **25.07.84 Patentblatt 84/30**

(84) Benannte Vertragsstaaten: **BE CH DE FR LI LU NL**

(56) Entgegenhaltungen:
**EP-A- 0 004 913**
**US-A- 3 994 933**
**JOURNAL OF THE AMERICAN CHEMICAL SOCIETY, Band 70, Nr. 3, 3. April 1948, Seiten 887-892 Washington D.C., U.S.A. PERCY L. JULIAN et al.: "Sterols. IV. delta 20-Pregnenes from bisnor-steroid acids"**

(73) Patentinhaber: **Henkel Kommanditgesellschaft auf Aktien**
**Postfach 1100 Henkelstrasse 67**
**D-4000 Düsseldorf-Holthausen (DE)**

(72) Erfinder: **Preuss, Wolfgang, Dr.**
**Finkenweg 12**
**D-4019 Monheim (DE)**
Erfinder: **Krbechek, Leroy Orville**
**2041 Orkla Drive**
**Golden Valley Minnesota 55427 (US)**

**EP 0 034 248 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die Erfindung betrifft die Umwandlung bestimmter Steroidverbindungen mit einer 20-Carboxylgruppe in die entsprechenden Carbonsäurehalogenidverbindungen und die dabei gewonnenen Produkte, die insbesondere wertvolle Zwischenprodukte für die Gewinnung von pharmakologisch aktiven Komponenten mit Steroidstruktur sind.

20-Carboxy-pregna-1,4-dien-3-on, das im folgenden der Kürze halber mit Δ1,4-BNC bezeichnet wird, hat bekanntlich die folgende Strukturformel :

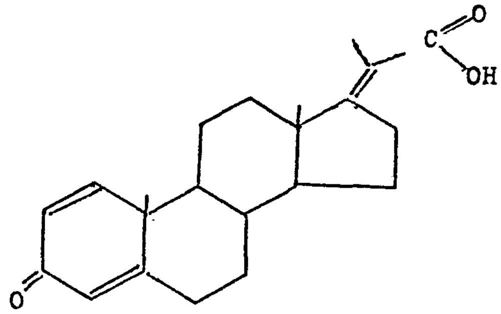

Verfahren zur großtechnischen Herstellung der Δ1,4-BNC auf mikrobiologischem Wege sind in der offengelegten europäischen Patentanmeldung 79101036.6 (4913) beschrieben. Eine weitere Ausgestaltung dieses Verfahrens wird in der offengelegten europäischen Patentanmeldung 79104165.0 (15308) geschildert. Δ1,4-BNC ist ein wertvolles Ausgangsmaterial zur Gewinnung verschiedenartigster Verbindungen der Steroidreihe. Als wünschenswert hat sich erwiesen, diese Carbonsäureverbindung in ein funktionelles Derivat umzuwandeln, das seinerseits als Ausgangsmaterial für eine Mehrzahl von Umsetzungen am Seitenkettenrest in 17-Stellung des Ringsystems dienen kann. Als solches funktionelles Derivat ist das der Δ1,4-BNC entsprechende Säurehalogenic geeignet. Für die Praxis haben insbesondere das Bromid und vor allen Dingen das entsprechende Δ1,4-BNC-Säurechlorid besondere Bedeutung.

In der österreichischen Patentanmeldung 582/80 (Anmeldedatum : 4.2.1980) der Anmelderin wird eine bis dahin nicht beschriebene, der Δ1,4-BNC strukturähnliche 20-Carboxy-Sterinverbindung beschrieben. Es handelt sich hierbei um das 20-Carboxy-pregna-1,4,17(20)-trien-3-on, das der Einfachheit halber als Δ1,4,17-BNC beschrieben wird. Diese Verbindung und Verfahren zu ihrer Herstellung sind gleichzeitig in der parallelen Patentanmeldung 81100146.0 (33439) (« Verfahren zur Herstellung von 20-Carboxy-pregna-1,4,17(20)-trien-3-on durch mikrobiellen Seitenkettenabbau an 17-C-Seitenketten-Steroidsubstraten ») geschildert. Δ1,4,17 hat die folgende Strukturformel :

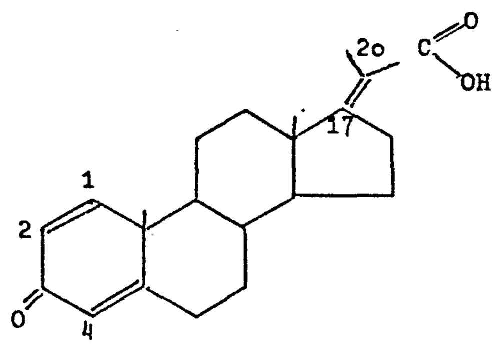

Die US-PS-3 994 933 beschreibt die Herstellung der 3-Oxo-pregna-4,17(20)dien-20-carbonsäure, die der Einfachheit halber als Δ4,17-BNC bezeichnet wird. Auch diese Säure wird durch mikrobiellen Seitenkettenabbau von 17 C-Steroidverbindungen gewonnen. Die Strukturformel dieser Verbindung ist die folgende :

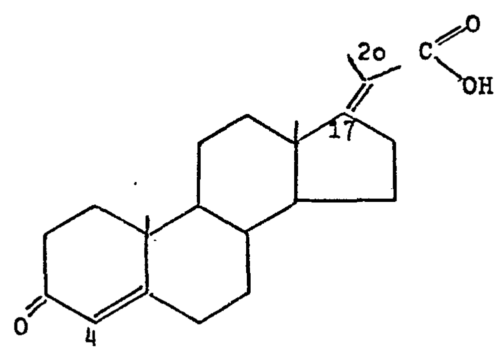

Auch bei den zuletzt geschilderten Δ4,17- und Δ1,4,17-BNC-Verbindungen besteht für die Fachwelt das Bedürfnis der Umwandlung dieser Carbonsäureverbindungen in ein funktionelles Derivat, das seinerseits Ausgangsmaterial für eine Mehrzahl von Umsetzungen am Seitenkettenrest in 17-Stellung des Ringsystems sein kann.

Die Überführung von Carbonsäuren in Säurechloride mittels anorganischer oder organischer Halogenierungsmittel ist eine an sich seit langem bekannte und allgemein benutzte Reaktion. So ist beispielsweise die Verwendung von Thionylchlorid oder Oxalylchlorid auch in der Reihe der 20-Carboxy-pregnan-derivate bereits vorgeschlagen worden.

Wendet man aber die in der Literatur — siehe hierzu beispielsweise Fiat Final Report No. 996, Seite 24 ff sowie P. L. Julian, E. W. Meyer und H. C. Printy, J. Amer. Chem. Soc. 70, 887 (1948) — für die Umsetzung von 3-Acetoxybisnorcholensäure mit Thionylchlorid angegebenen Reaktionsbedingungen auf Δ1,4-BNC an, verestert danach das so erhaltene Säurechlorid mit Methanol und analysiert das Rohprodukt, so findet man im Gaschromatogramm einen zusätzlichen Peak und bei der Elementaranalyse einen deutlichen, zunächst nicht erwarteten Cl-Gehalt. Ähnliches gilt sogar in noch stärkerem Maße für die Verwendung von Oxalylchlorid anstelle von Thionylchlorid.

Der Grund für das Auftreten dieser unerwünschten Verunreinigungen, die die Ausbeute an gewünschtem Produkt deutlich senken und zu Reinigungsproblemen bei weiteren Umsetzungen mit dem Säurechlorid führen können, ist wahrscheinlich eine Chlorierung mit eventuell anschließender Aromatisierung des A-Ringes im Steroidgerüst, wie sie z. B. für die Umsetzung von Androsta-1,4-dien-3,17-dion (ADD) mit Oxalylchlorid bekannt ist ; siehe hierzu G. W. Moersch et al., J. Org. Chemistry, 29, 2495 (1964).

Vergleichbare Schwierigkeiten treten auf, wenn nach der bisher bekannten Verfahrenstechnik Δ1,4,17-BNC oder Δ4,17-BNC als Ausgangsmaterial eingesetzt werden.

Die sorgfältige Durcharbeitung der Reaktion zwischen den erfindungsgemäß betroffenen mehrfach ungesättigten 20-Carboxy-pregnan-verbindungen mit Thionylhalogenid, insbesondere mit Thionylchlorid, erbrachte das überraschende Ergebnis, daß die Bildung des gewünschten Säurechlorids der mehrfach ungesättigten Ausgangsverbindung bereits unter außergewöhnlich milden Reaktionsbedingungen erfolgen kann, bei denen die unerwünschte Mitreaktion anderer reaktiver Stellen der mehrfach ungesättigten BNC-Struktur noch nicht stattfindet. So zeigte sich das überraschende Ergebnis, daß eine praktisch quantitative Säurechloridbildung stattfindet, wenn die folgenden Reaktionsbedingungen eingehalten werden : Reaktionstemperature, unterhalb 10 °C, vorzugsweise unter 5 °C, stöchiometrische Mengen der Reaktanten oder nur sehr begrenzter Überschuß des Thionylchlorids, der nicht über 20 Molprozent, vorzugsweise nicht über 10 Molprozent beträgt, sowie kurze Reaktionszeiten, die vorzugsweise 30 Minuten nicht überschreiten. Als Umsetzungsbedingungen ist insbesondere die folgende Kombination geeignet : etwa 0 °C, Thionylchloridüberschuß von 0-10 Molprozent und Reaktionsdauer von 15-20 Minuten in Gegenwart eines inerten Lösungsmittels.

Gegenstand der Erfindung sind dementsprechend in einer ersten Ausführungsform die neuen bisher nicht beschriebenen Δ4- sowie wenigstens eine weitere Doppelbindung in 1(2)- und/oder 17(20)-Stellung aufweisenden Pregna-3-on-20-carbonsäurehalogenide der allgemeinen Formel :

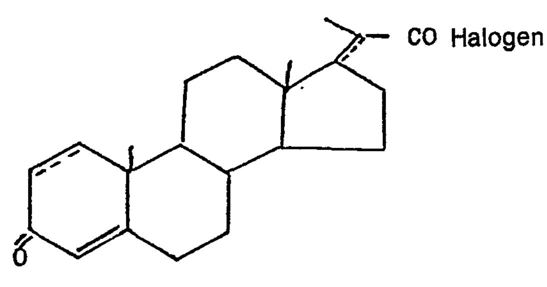

(I)

In dieser Formel bedeutet Halogen insbesondere Chlor oder Brom, wobei dem Chlor erfindungsgemäß die wichtigste Bedeutung zukommt. Die Erfindung betrifft damit in ihrer bevorzugten Ausführungsform das Δ1,4-BNC-Chlorid, das Δ1,4,17-BNC-Chlorid und das Δ4,17-BNC-Chlorid. Die beiden zuerst genannten Säurechloride sind die erfindungsgemäß wichtigsten Verbindungen.

Die Erfindung betrifft in einer weiteren Ausführungsform das Verfahren zur Herstellung dieser neuen mehrfach ungesättigten Pregna-3-on-20-carbonsäurehalogenide unter den zuvor geschilderten Reaktionsbedingungen, die die selektive Umwandlung der 20-Carboxylgruppe in die entsprechende Carbonsäurehalogenidgruppe ermöglichen ohne daß an sich reaktionsbereite weitere Stellen des mehrfach ungesättigten Steroidgerüsts in substantiellem Ausmaß mit-reagieren.

Ein wichtiges Element dieses Verfahrens ist die Auswahl des geeigneten Halogenierungsmittels. Wenn auch allgemein anorganische Halogenierungsmittel — neben den Thionylhalogeniden beispielsweise Phosphorhalogenidverbindungen — in Betracht gezogen werden können, so kommt dem Thionylhalogenid doch die überragende Bedeutung zu. Geeignete Thionylhalogenide sind insbesondere Thionylchlorid, Thionylbromid oder Thionylfluorid. Für die Zwecke der Erfindung sind die beiden zuerst genannten Verbindungen die wichtigsten, hiervon wieder ist das Thionylchlorid die bevorzugte Wahl des erfindungsgemäßen Verfahrens. Die Umsetzung wird vorzugsweise im Temperaturbereich um 0 °C

durchgeführt, wenngleich allgemein gilt, daß Temperaturen von etwa – 20 °C bis etwa + 20 °C zur Verwendung kommen können. Zum sicheren Ausschluß unerwünschter Nebenreaktionen wird bei Temperaturen unter 10 °C, vorzugsweise unter 5 °C gearbeitet.

Das Halogenierungsmittel wird erfindungsgemäß in stöchiometrisch erforderlicher Menge oder äußerstenfalls in sehr geringem Überschuß eingesetzt. Dieser Überschuß beträgt zweckmäßig nicht mehr als 20 Molprozent und vorzugsweise nicht mehr als 10 Molprozent.

Es ist weiterhin bevorzugt die Umsetzung in Gegenwart eines Lösungsmittels durchzuführen, das gegenüber der eingesetzten Steroidverbindung, dem Halogenierungsmittel und dem entstehenden Säurehalogenid inert ist. Geeignet als inerte Lösungsmittel sind beispielsweise halogenierte Kohlenwasserstoffe, wie Methylenchlorid, Chloroform oder Ethylendichlorid geeignet. Mit Einschränkungen können auch Ether in Betracht kommen.

Katalytische Mengen einer Base, insbesondere Pyridin oder Dimethylformamid können erfindungsgemäß mitverwendet werden. Charakteristisch für die Umsetzung der Erfindung unter den angegebenen Bedingungen ist jedoch, daß grundsätzlich kein Zusatz eines Katalysators und kein Zusatz einer größeren Mente an Base, wie beispielsweise tertiäres Amin oder Alkalicarbonat, erforderlich ist. Bevorzugt wird ohne Zusatz eines Katalysators gearbeitet.

Die Reaktion wird üblicher Weise bei Normaldruck durchgeführt. Das Halogenierungsmittel wird zweckmäßigerweise zu der Lösung der umzusetzenden Steroidverbindung im inerten Lösungsmittel gegeben. Es hat sich als vorteilhaft erwiesen, das Halogenierungsmittel in möglichst reiner Form einzusetzen. Offenbar fördern üblicherweise im Halogenierungsmittel vorliegende Verunreinigungen unerwünschte Nebenreaktionen. Zweckmäßig kann beispielsweise eine Reinigung des Halogenierungsmittels mit einer ungesättigten Verbindung, wie Leinöl oder besonders Squalen, sein. Diese ungesättigten Komponenten reagieren mit den Verunreinigungen im Halogenierungsmittel und senken damit die Bildung unerwünschter Nebenprodukte.

Die erfindungsgemäßen BNC-Halogenide der eingangs angegebenen allgemeinen Formel I und insbesondere das Δ1,4-BNC-Chlorid (Pregna-1,4-dien-3-on-20-carbonylchlorid), das Δ1,4,17(20)-BNC-Chlorid (Pregna-1,4,17(20)-trien-3-on-20-carbonylchlorid) und das Δ4,17(20)-BNC-Chlorid (Pregna-4,17(20)-dien-3-on-20-carbonylchlorid) sind bisher in der Literatur nicht beschriebene Verbindungen. Diese Säurechloride eignen sich als Ausgangsmaterial für nachfolgende Reaktionen zur weiteren strukturellen Umwandlung des Seitenkettensubstituenten in 17-Stellung des Steroidringgerüsts.

So können z. B. in an sich bekannter Weise durch Umsetzung der Säurehalogenide mit Alkoholen — gewünschtenfalls in Gegenwart von säurebindenden Mitteln wie tertiären Aminen — die entsprechenden Ester der eingesetzten BNC-Verbindungen erhalten werden. Als Alkohole eignen sich beispielsweise wasserfreie Alkanole, wie Methanol, Ethanol, Propanole und Butanole einschließlich tert. Butanol. Als säurebindende Komponenten kommen die bereits erwähnten tertiären Amine, wie Triethylamin oder Pyridin in Betracht. Geeignet sind aber auch säurebindende anorganische Salze, beispielsweise Carbonate wie Calciumcarbonat oder Caliumcarbonat. Die basischen Verbindungen können in äquimolaren Mengen, gewünschtenfalls aber auch im Überschuß oder auch im Unterschuß eingesetzt werden. Die Umsetzung erfolgt im allgemeinen bei höchstens schwach erhöhten Temperaturen, z. B. im Temperaturbereich von – 10-30 °C. Es kann in Gegenwart von inerten Lösungsmitteln oder vorzugsweise in einem Überschuß des Alkohols als reaktives Lösungsmittel gearbeitet werden.

Die Herstellung der neuen BNC-Halogenidverbindungen, insbesondere der entsprechenden BNC-Chloride und ihre analytischen Daten sind in den folgenden Beispielen beschrieben :

## Beispiel 1

17 g (50 mMol) Δ1,4-BNC in 100 ml absoluten $CH_2Cl_2$ werden bei 0 °C mit 4,0 ml (55 mMol) frisch über Squalen destilliertem Thionylchlorid versetzt und 20 Minuten bei 0 °C gerührt. Danach werden das Lösungsmittel und überschüssiges Thionylchlorid bei der gleichen Temperatur im Vakuum entfernt. Der Rückstand wird wieder in Methylenchlorid aufgenommen und die Lösung nochmals zur Trockene eingeengt. Der Rückstand ist für weitere Umsetzungen zu verwenden. Um ein zur Analyse geeignetes Säurechlorid zu erhalten, wird das rohe Säurechlorid mit absolutem Ether digeriert und nach dem Abziehen des Ethers sorgfältig an der Ölpumpe getrocknet.

Elementaranalyse

| | | | |
|---|---|---|---|
| ber. | C 73,2 | H 8,10 | Cl 9,82 |
| gef. | C 73,0/72,9 | H 8,01/7,95 | Cl 9,72/9,51 |

Die Chlorid-Bestimmung nach Hydrolyse einer Probe ergab 9,75 % Chlor als $Cl^-$ (berechnet 9,82 %).
Smp. (des rohen Säurechlorids) : 142 °C Zersetzung, nach Sintern bei 135° bis 138 °C.

## Beispiel 2

17 g (50 mMol) Δ1,4,17-BNC in 100 ml absoluten $CH_2Cl_2$ werden bei 0 °C mit 4,0 ml (55 mMol) frisch

über Squalen destilliertem Thionylchlorid versetzt und 20 Minuten bei 0 °C gerührt. Danach werden das Lösungsmittel und überschüssiges Thionylchlorid bei der gleichen Temperatur im Vakuum entfernt. Der Rückstand wird wieder in Methylenchlorid aufgenommen und die Lösung nochmals zur Trockene eingeengt. Der Rückstand ist für weitere Umsetzungen zu verwenden. Um ein zur Analyse geeignetes Säurechlorid zu erhalten, wird das rohe Säurechlorid mit absolutem Ether digeriert und nach dem Abziehen des Ethers sorgfältig an der Ölpumpe getrocknet.

Elementaranalyse

ber. C 73,2 H 8,10 Cl 9,82
gef. C 72,85 H 8,22 Cl 10,1

Die Spektraanalyse liefert die folgenden Werte :
$^1$H-NMR-Spektrum (90 MHZ, $CDCl_3$, Δ-Werte) :

18-$CH_3$ : 1,01 ppm · s
19-$CH_3$ : 1,24 ppm · s
21-$CH_3$ : 2,07 ppm · t (I = 1,9 Hz)

— olefinische Protonen : ABC-System mit Linien bei

6,07 ; 6,15 ; 6,17 ; 6,26 ;
6,28 ; 7,00 ; 7,11 ppm

— die restlichen Protonen liefern Signale im erwarteten Bereich.

Beispiel 3

5 g (14,6 mmol) Δ1,4-BNC in 20 ml trockenem Methylenchlorid versetzt man bei 0 °C mit 3,34 g (16,1 mmol) frisch im Vakuum destilliertem Thionylbromid.

In Abständen von 10 Minuten wurden Proben entnommen und der Fortgang der Reaktion nach Veresterung mit Methanol/Pyridin auf der Dünnschicht verfolgt. Beurteilt wurde das Verhältnis BNC-Methylester/BNC. Nach 30 Minuten war die Umsetzung zum Säurebromid praktisch vollständig.

Durch Umsetzung des rohen Säurebromids mit einem Überschuß Methanol/Pyridin und Isolierung des Produkts nach üblichen Methoden wurden 4,4 g BNC-Methylester erhalten, der nach IR- und NMR-Spektrum sowohl mit einem auf die gleiche Weise aus Δ1,4-BNC-Chlorid als auch mit einem durch Umsetzung von Δ1,4-BNC mit Diazomethan erhaltenen Ester identisch war.

Der Schmelzpunkt des aus Δ1,4-BNC durch Veresterung mit Diazomethan erhaltenen Esters lag bei 174-176 °C.

**Ansprüche**

1. Δ4- sowie wenigstens eine weitere Doppelbindung in 1(2)- und/oder 17(20)-Stellung aufweisende Pregna-3-on-20 carbonsäurehalogenide (BNC-Halogenide) der allgemeinen Formel

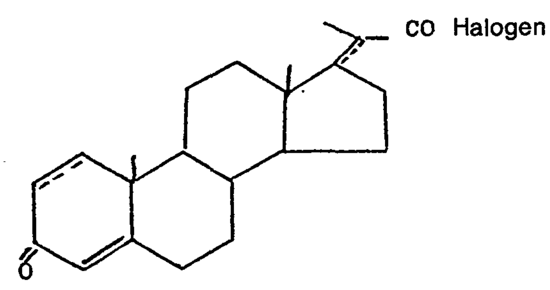

(I)

2. Δ1,4-BNC-Chlorid.
3. Δ1,4,17-BNC-Chlorid.
4. Verfahren zur Herstellung von Δ4- sowie wenigstens eine weitere Doppelbindung in 1(2)- und/oder 17(20)-Stellung aufweisenden Pregna-3-on-20-carbonsäurehalogeniden nach Anspruch 1, dadurch gekennzeichnet, daß man die der allgemeinen Formel I entsprechende Pregna-3-on-20-carbonsäure mit Thionylhalogenid in stöchiometrischer Menge oder einem Überschuß des Thionylhalogenids nicht über 20 Mol-% bei Temperaturen nicht über 10 °C, vorzugsweise unter 5 °C bei einer maximalen Reaktionszeit von 30 Minuten umsetzt.

5. Verfahren nach Anspruch 4, dadurch gekennzeichnet, daß man in einem inerten Lösungsmittel, beispielsweise in halogenierten Kohlenwasserstoffen, wie Methylenchlorid, arbeitet.

6. Verfahren nach Ansprüchen 4 und 5, dadurch gekennzeichnet, daß man mit Thionylchlorid arbeitet und dieses in einem molaren Überschuß von 0 bis 10 % einsetzt.

7. Verfahren nach Ansprüchen 4 bis 6, dadurch gekennzeichnet, daß man die Reaktion bei annähernd 0 °C durchführt und nach 15 bis 20 Minuten beendet.

8. Verfahren nach Ansprüchen 4 bis 7, dadurch gekennzeichnet, daß man mit einem über ungesättigten Verbindungen wie Leinöl oder Squalen vorgereinigten Thionylchlorid arbeitet.

**Claims**

1. Δ4-pregna-3-one-20-carboxylic acid halides (BNC-halides) containing at least one other double bond in the 1(2)- and/or 17(20)-position and corresponding to the following general formula

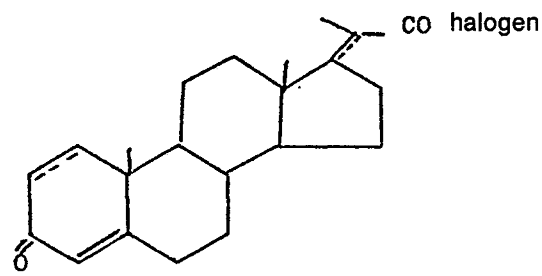

(I)

2. Δ1,4-BNC-chloride.

3. Δ1,4,17-BNC-chloride.

4. A process for producing the Δ4-pregna-3-one-20-carboxylic acid halides containing at least one other double bond in the 1(2)- and/or 17(20)-position claimed in Claim 1, characterized in that the pregna-3-one-20-carboxylic acid corresponding to general formula I is reacted with the stoichiometric quantity or with an excess of no more than 20 mole percent of thionyl halide for at most 30 minutes at temperatures not exceeding 10 °C and preferably at temperatures below 5 °C.

5. A process as claimed in Claim 4, characterized in that the reaction is carried out in an inert solvent, for example in halogenated hydrocarbons, such as methylene chloride.

6. A process as claimed in Claims 4 and 5, characterized in that thionyl chloride is used and is employed in a molar excess of from 0 to 10 %.

7. A process as claimed in Claims 4 to 6, characterized in that the reaction is carried out at approximately 0 °C and is terminated after 15 to 20 minutes.

8. A process as claimed in Claims 4 to 7, characterized in that a thionyl chloride prepurified through unsaturated compounds, such as linseed oil or squalene, is used.

**Revendications**

1. Halogénures de Δ4- ainsi que des halogénures d'acide prégna-3-one-20-carboxylique (halogénures de BNC) comportant au moins une autre double liaison en position 1(2)- et/ou 17(20) répondant à la formule générale

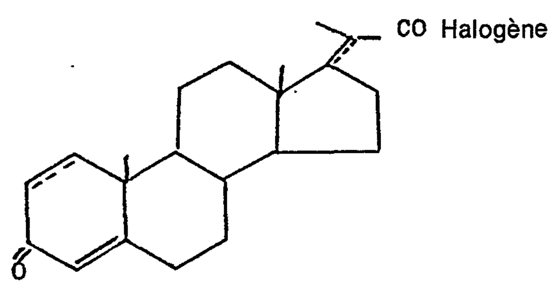

(I)

2. Chlorure de Δ1,4-BNC.

3. Chlorure de Δ1,4,17-BNC.

4. Procédé pour la préparation d'halogénures de Δ4- ainsi que d'halogénures d'acide prégna-3-one-20-carboxylique comportant au moins une autre double liaison en position 1(2) et/ou 17(20) selon la revendication 1, caractérisé en ce qu'on fait réagir l'acide prégna-3-one-20-carboxylique répondant à la formule générale I avec de l'halogénure de thionyle en quantité stœchiométrique ou avec un excès de

l'halogénure de thionyle ne dépassant pas 20 % molaire, à des températures non supérieures à 10 °C, de préférence inférieures à 5 °C pendant une durée de réaction maximale de 30 minutes.

5. Procédé selon la revendication 4, caractérisé en ce qu'on opère dans un solvant inerte, par exemple dans des hydrocarbures halogénés tels que le chlorure de méthylène.

6. Procédé selon les revendications 4 et 5, caractérisé en ce qu'on opère avec du chlorure de thionyle et le met en œuvre avec un excès molaire de 0 à 10 %.

7. Procédé selon les revendications 4 à 6, caractérisé en ce qu'on effectue la réaction à environ 0 °C et la termine après 15 à 20 minutes.

8. Procédé selon les revendications 4 à 7, caractérisé en ce qu'on opère avec du chlorure de thionyle préalablement purifié sur des composés insaturés tels que l'huile de lin ou le squalène.